# EUROPEAN PATENT APPLICATION

(11) **EP 2 116 214 A1**
(43) Date of publication of application: **11.11.2009**
(21) Application number: 08156013.8
(22) Date of filing: 09.05.2008
(51) Int. Cl.: A61F 5/08, A61F 5/56

(54) **A nasal device useful for prevention of snoring and sleeping apnea**

(71) Applicant: Al-Zeir, Nasri Hasan Moh'd, 11953 Tla-Al-Ali, Amman (JO)
(72) Inventor: Al-Zeir, Nasri Hasan Moh'd, 11953 Tla-Al-Ali, Amman (JO)
(74) Representative: Viering, Jentschura & Partner

(57) **Abstract**

The present invention deals with a nasal device which is useful for prevention of snoring wherein the present device works on keeping the nares normally open mechanically, by abducting the nasal vestibule (upper & anterior portion of the lateral wall of the nose which is covered by skin), forward and laterally, which makes air to flow in and out adequately. A stainless steel skelton having one continuous stainless steel wire of unique design (as apparent in Figure 3), configured with the spring (1) having two prominent side knobs (2) and two resilient acute angles (6) to permit the abduction arms (4) and plates (7) to be worn (or inserted) inside the nose, wherein the said arms consisting of abduction plates (7) at its free end. Its base resting on the columellae outside the nose which is in continuity with the abduction arms by the said base arms (3), and the whole said device is coated with a thin layer of latex sponge or silicon to make it comfortable during use.

## Description

### FIELD OF INVENTION:

The present invention relates to the nasal device and more particularly the said nasal device may be used as a nasal guard for preventing snoring and sleeping apnea.

### BACKGROUND AND PRIOR ART OF THE INVENTION:

Snoring is a big and universal medico- social complaint. According to the available statistics it could affect more than 70% of adult males and more than 30% of adult females being all over the world. (American health statistics 2006) Normally in man the pressure inside the nose fluctuates within 10 to 15 (+6 to -6) mm water. This fluctuation mainly depends on the condition of the nares (external opening of the nose), and the normality of air flow.

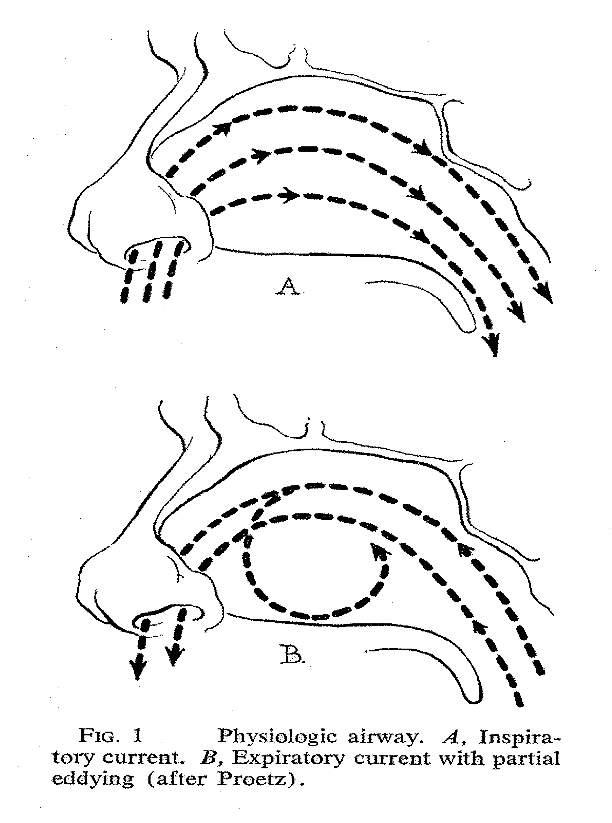

When nares are obstructed partially or totally due to any one of the many causes, the pressure equilibrium between anterior and posterior nasal chambers fluctuates and the soft palate is pulled by vacuum mechanism (Figure 1) upwards to close the nasopharynx passage or chonae (posterior nasal opening), due to which the mouth reflexly opens, and breathing turns to mouth. The passage of air in & out through mouth creates the harsh or rustling noise, which is known as snoring.

The following prior art references have been considered in entirety to be similar in respect of the present invention. However none of the prior art discloses the features of the present invention.

US 7,080,645 describe an anti-snoring device comprising a compressor and a nasal air cannula, the air compressed by the compressor being blown through the nasal air cannula into the nose of a sleeping person. The invention also relates to an optimized nasal air cannula for the anti-snoring device. This invention is a different; because it is comprised of a big machine with connections out side the nose. Also it needs source of energy to function. The cannula and connections makes it to interfere with the sleep.

DE102005037843 describes the particular circumstances interfering with the nasal breathing leading to snoring. A person could have very narrow nostrils, the nasal mucosa could temporarily release excess secretion, the nostrils could be generally weak and the inner surfaces cling to each other. These problems can be solved with two small spirals to be inserted into the nostrils and worn during the night. The spirals are made of stainless steel and can be coated with a skin-friendly material. This device is made to deal with one cause of snoring (narrow nostrils), while there are more than one hundred causes for snoring; Physiological and pathological. The second difference it works on separation mechanism; by separating the two sides of nostrils, which could harm the nasal septum if it is too weak.

KR100257899B deals with snoring prevention and nasal correction device. This is exclusively a nasal correction device, used to aid nasal surgery specially in case of SMR.

TW541173B relates to a method for producing a device for curing snoring and its product, which mainly consists of providing a clamp for easily clamping the fore front of the nasal septum of a user for generating the stimulation on the nasal septum in order to tighten the soft midriff enabling smooth breathing. As a result, the snoring caused by a relaxed soft midriff in sleep can be avoided. The method comprises: installing a horse-shoe-shaped frame with a slightly inward convergence on its two ends; injecting a soft plastic to wrap around the frame; mounting a plurality of protruded blocks on the coated frame to stably hold the frame such that the coated frame will not shift or tilt over during the coating process, thereby increasing the yield. This invention is also designed to deal with one cause of snoring (relaxed soft midriff). It is built on mechanism of stimulating the nerve endings. It is completely different from the present invention.

US2007277832 describes the nasal respiratory devices and methods for treating a variety of medical diseases including snoring and sleep apnea through the use of such devices. In general, these devices include an airflow resistor, such as a flap valve and a holdfast for securing the device in communication with the subject's nasal cavity. The devices may be configured to include leak paths to regulate the expiratory pressure when worn by a subject. Methods for using these devices may include securing a device over or at least partially within (or both of) a subject's nasal cavities.

It is not made exclusively to deal with snoring (but for variety of diseases). It has a mask over the nose with connection to a machine situated out side the nose. It is different in mechanism, size, shape, complexity and etc.

WO0066048 deals with a device for improving respiration and eliminating snoring, comprising elements for fixing to a portion of the face of a person and by engagement elements for engaging two regions of the nose which are spaced from the nasal lobule or columella and are arranged on opposite sides with respect to the nasal lobule; the engagement elements are connected to the fixing means through at least one traction element, so that during the traction of the grip region a simultaneous effect of lifting the columella and of lateral rotation inside the nose is achieved to dilate the nostrils of the nose along their entire extent and allow better passage of the air in the nasal cavities. However, it works by pulling ( traction) of the nose upwards and outside. It has connection to other part of the body (face). This is a different mechanism and different in shape, size and place of fixing.

WO2005051253 relates to a device for preventing snoring. The device is in the form of U-shape, two ends of the U-shape can be put into nostrils as a holder. Magnetic pieces are embedded into the ends of the U-shape, so as to maintain the holder onto nasal septa by the magnetic force. Furthermore, the magnetic field generated by the magnetic pieces can stimulate the nasal septa and trigeminal ending, so as to prevent constriction of air passage and keep the air passage open. The present device is more available than conventional one in compression form. This invention has been built on magnetic mechanism, which has a stimulating effect on nerve endings. It is not stable therefore it could slip inside or outside of the nose by movement during sleep. It differs a lot from the invention under subj ect.

WO2005107662 relates to an anti-snoring nasal device comprising two hooks fixed to a single support and means fixable to a patient head and slightly pulling in the direction of the patient forehead, wherein said fixing and pulling means consists of a flexible adhesive strip applicable to the patient forehead above the nose base and of a flexible thin tie connecting said strip to the single support, said device also comprises an element which is made of biocompatible material and introducible between said flexible thin tie and the forehead or the nose root area in such a way that the tie is remote and the vertical pulling direction of the tie is brought closer to the nose. This is also a different entity. It depends on traction mechanism like the invention above (WO0066048). It is also for out side nasal use. Therefore, it is far different from the under subject.

WO2007141492 deals with a device for alleviating snoring comprising first and second substantially spherical parts, the first and second spherical parts being insertable into a first and a second nasal passage respectively of a user, wherein, in use, air flow through the nasal passages of the user is substantially restricted.

Most of the above referred prior art devices has a component which is incorporated inside the nostrils for prevention, elimination or curing of snoring. This is using restriction of air flow mechanism whish is different from the under subject. Both parts of it are not in continuity, which could expose the user to the danger of the device to slip in or out side the nose during sleep.

### OBJECTS OF THE INVENTION:

The main object of the invention is to provide a nasal device for preventing snoring & sleeping apnea in an effective manner.

An object of the invention is to provide a nasal device which totally rests on the vestibule and adapts it self according to the opening size of the nose.

Another object of the invention is to provide a nasal device which is very small in size, simple, easy and safe to use, washable and can be sterilized.

Further, object of the invention is to provide a nasal device which avoids its use in the sensitive mucous membrane and stable in its place despite movements of the user during sleep.

### SUMMARY OF THE INVENTION:

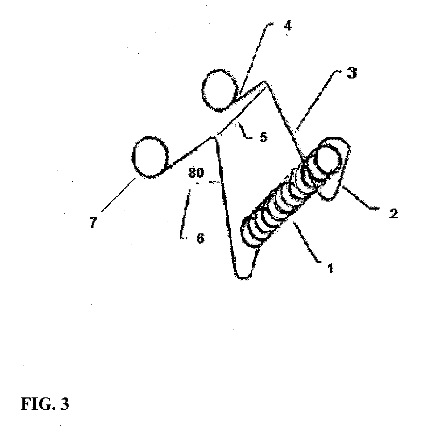

The present invention relates to a nasal device useful for prevention of snoring and sleeping apnea, comprising a stainless steel Skelton having at least one continuous stainless steel wire of peculiar design (as shown in figure 3), configured with the spring having two prominent side knobs and two resilient acute angles to permit the abduction arms designed to be worn inside the nose, wherein the said arms consisting of abduction plates at its free end. Its base rests on the colamellae outside the nose and the said device is coated with a thin layer of latex sponge or silicon (for those who have history of allergy to Latex) to make it comfortable during use.

### BRIEF DESCRIPTION OF THE FIGURES:

Figure 1 represents currents of breathing during inspiration and in case of expiration the pressure reverses.
Figure 2 represents the pressure equilibrium between anterior and posterior nasal chambers fluctuates and the soft palate is pulled by vacuum mechanism.
Figure 3 represents the present nasal device and its components, where each reference numerals indicate the following components:
   1- Spring
   2- Side knobs
   3- Base arms
   4- Abduction arms
   5- Internal opening
   6- Base abduction angles
   7- Abduction plates
Figure 4 represents the present nasal device coated.
Figure 5 represents the present nasal device in use

### DETAILED DESCRIPTION OF THE INVNETION:

Accordingly the present invention provides A nasal device useful for prevention of snoring and sleeping apnea, wherein the said device comprising a stainless steel Skelton having one continuous stainless steel wire of unique design (as apparent in Figure 3), configured with the spring (1) having two prominent side knobs (2) and two resilient acute angles (6) to permit the abduction arms (4) and plates (7) to be worn (or inserted) inside the nose, wherein the said arms consisting of abduction plates (7) at its free end. Its base resting on the columellae outside the nose, which is in continuity with the abduction arms by the said base arms (3), and the whole said device is coated with a thin layer of latex sponge or silicon to make it comfortable during use.

In an embodiment of the present invention, the said stainless steel wire used is having a diameter of at least about 0.7mm.

In another embodiment of the present invention, the length of the abduction arm and its plate( as one unit), is in two sizes (1.6mm & 1.4mm long each) to fit in different noses.

Yet another embodiment of the present invention, the length of base arms are about 10 mm to facilitate controlling abduction arms.

Still yet another embodiment of the present invention, the internal opening of the device enlarges by pressing of the side knobs.

Further embodiment of the present invention , the resilient acute angles have about 80 degree each.

Still in another embodiment of the present invention, the abduction plates at the free ends of the abduction arms, in a circle form about 3mm diameter.

Still yet another embodiment of the present invention, the latex sponge and silicone coating used in a thickness of at least about 1mm.

The present invention "Anti snoring Nasal Guard" (ASNG), is a small mechanically working device has been invented to exclusively deal with the big medico-social problem of snoring during sleep, which is the cause of suffering to majority of adults and their companions, all over the world.

The "ASNG" has been designed to break through the snoring formation mechanism through inhibiting marked fluctuation in the normal pressure of the anterior and posterior nasal chambers, by maintaining the normal function of the anterior nasal air passage through the anterior nares.

To explain how "ASNG" works, It is necessary to explain first how snoring happens refer to page 1).

Therefore, the ASNG device performs its job by breaking through the chain of snoring producing mechanism explained above, through maintaining normal pressure equilibrium between anterior & posterior nasal chambers.

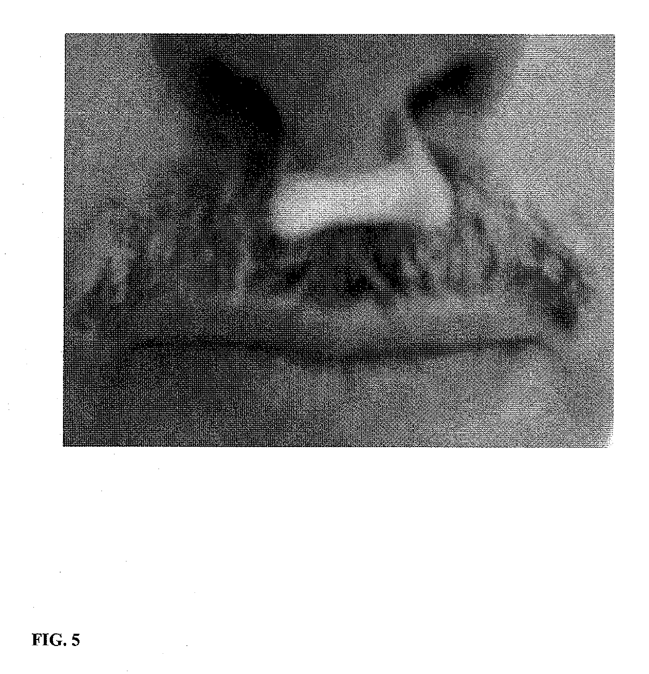

As it is apparent from the Figures. 3, 4 & 5, that the "ASNG" device is unique in its shape and design. Intended to do its job in a perfect manner, as its whole body rests on the vestibule (the anterior parts of the nose lined by skin), instead of the sensitive mucous membrane.

This is in addition to its two different sizes of abduction arms which have been designed to fit properly in different noses without extra enlarging the nostrils and sake of preventing side effects such as dryness and crusting of nasal mucous membrane lining the breathing passages.

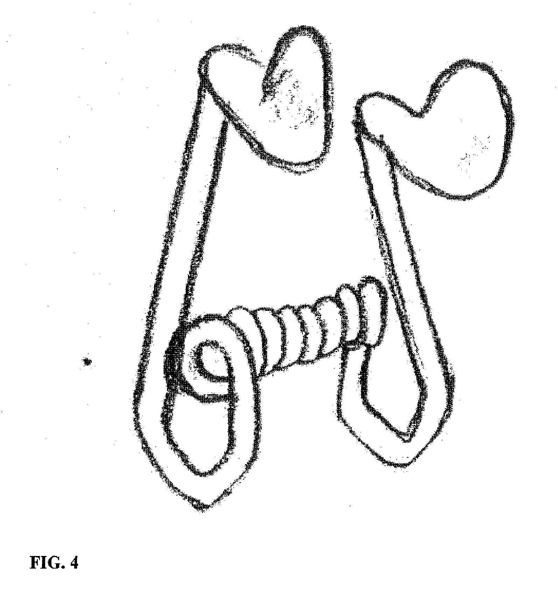

The "ASNG" device is distinguished from other anti snoring already invented devices, by the following elements:
(A) It has proved to prevent snoring in more than 90% of users, and to prevent sleeping apnea in more than 60% which is evidenced by example 1.
(B) As it is clear in Figures 3 & 4, that the device under discussion has a spring (part 1) at its base. The spring's main functions are; First function is to adapt the size of the opening (part 5), according to size of the nasal columella of the user.
   Second function is the adequate resilience it gives to both abduction arms (part 4) of the device, which allow the lateral wall of nose to move in and out freely as in normal person.
   The third, it makes wearing and taking off the device easier and safer to the user.
   The forth function of the spring, is to keep the device fixed in its place despite the movements of the user during sleep.
(C) Its stainless steel skeleton, Figure 3 and its acute angle 80 degree (part 6), which allow its arms (part 4) when inside nostrils, to bend forward to rest against the nasal vestibule ( the area of nose covered by skin) to avoid side effects which could occur if the device rests on the mucous membrane of the nose directly.
(D) It is also comprised of two abduction arms (4), at the free end of each there is an abduction plate (7) of circular form (of 3mm diameter).
   The abduction arm and its plate length, is made of two sizes (14mm & 16 mm long), to fit in side different noses (big and small noses). Because these are the two parts of the device inside the nose responsible for abduction mechanism, and are designed to give enough resilience to the lateral nasal wall to move freely in and out as in normal nose.
(E) The base of the device is in continuity with abduction arms through the two base arms (3), which maintain the device in place and form the internal opening of the device.
(F) It is comprised of one continuous stainless steel wire of 0.7mm diameter. It gives the device stability and resilience in place, (Figure 3), as well as it reduces its weight.
(G) Its unique design (shown in Figure 3&4) which allows it to be opened and closed during wearing and removal smoothly by gentle pressing on the side knobs (2), without causing any pain or harm to the nasal soft tissue or affecting its stability.
(H) It is also covered completely by a thin layer of Latex sponge or silicon for those people has history of allergy to latex (Figure 4). This cover makes it friendly and comfortable during use and allows the device to be washed by water and soap, as well as it can be sterilized in Autoclave.
(I) The mechanism: As mentioned earlier the pressure inside nose of a normal person fluctuates within 10 - 15 (+6 to -6) mm water. This fluctuation mainly depends on the status of nares (External opening of the nose),

Therefore, the present device (ASNG) works on keeping the nares normally open mechanically, by abducting the nasal vestibule (upper & anterior portion of the lateral wall of the nose which is covered by skin), forward and laterally, which makes air to flow in and out adequately. This correction reverses the effect of obstruction of the nares, by equalizing the pressure in the anterior and posterior champers of nose which makes the soft palate to restore its normal position, and mouth closes and air flows normally in & out through the nasal passages, and thence snoring does not happen.

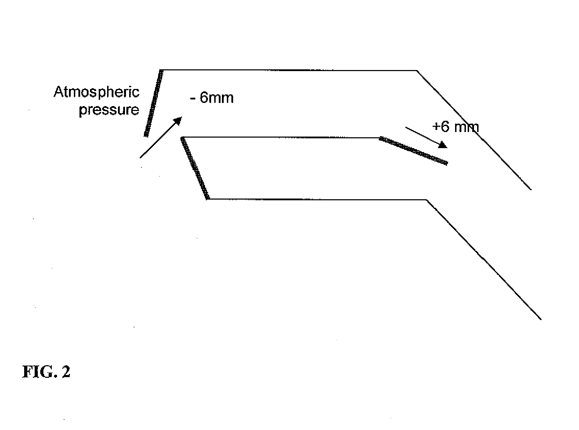

Its evident mechanism which was based on scientific research to explain how snoring happens, as was discussed earlier in the introduction. It is also the only device built to break through snoring formation cycle depending on Vacuum theory which is explained by in the figure 2:
Figure 2 shows currents of breathing during inspiration and in case of expiration the pressure reverses. And when any marked fluctuation in pressure happens due to closure of the anterior nares, vacuum power pulls the soft palate upwards, and mouth reflexly opens.

The following examples are an illustrative of the present invention and not to be construed to restrict the invention.

### EXAMPLES

### Executive Summary of the study on efficiency of the device

A study on 20 snoring adult men was carried out during Nov. 2007.

They were selected randomly (every second one according to their arrival to the office), out of 43 adult snoring volunteers responded to our request for trying the device. They were divided into five groups (because only 4 prototype devices were available). Each volunteer used it during sleep at his own home for 5 continuous days.

A questionnaire of two parts was answered by each one of the sample members. The first part contained 6 questions were filled before use and second part comprised of 4 questions has been completed after 5 days of continuous use of the device during sleep.

All before hand, were requested to fill a form includes their consent to participate in this trial, confidentiality commitment, and oath of tilling the truth.

### Results of pre- use, were as follows:

- **Social history:** All the 20 men were married male, suffering from snoring during sleep and their wives frequently complain of disturbance and some of them move to sleep in another place. All of them admitted that snoring is a real problem to them.
- **Age group:** They were of the following age groups; 3 of them belonged to 20-39 years old group; 12 (60%) were of 40-59 years group and only 5 belonged to above 60 years old group.
- **Symptoms of snoring:** 18 of them complained of dry mouth when they get up from sleep, 10 complained of dry mouth and being tired when they get up from sleep. 7 complained of palpitation, dry mouth and tired. One man complained of nervousness in addition to dry mouth, palpitation and night mares. Two only did not complain of any symptom.
- **Diseases:** Out of the sample 5 were suffering from cardiovascular diseases, 3 were diabetic, 2 sleeping apnea and one was a patient of bronchial asthma.
- **Nasal Diseases:** Regarding nasal pathologies they were as follows: 6 of them gave history of allergic rhinitis, 2 polyps; one hypertrophied turbinate or deviated nasal septum (DNS). And the other 11 gave no history.

### After 5 days of using the device, results were as follows:

- **Satisfaction:** 19 persons (95%), of them were satisfied and their wives admitted that they slept well. 16 of the wives said that snoring stopped completely and 3 wives said snoring stopped by about 75% only. One wife said she did not feel much change, her husband one of those who had history of polyp.
- **Fitting status;** 2 only complained that some nights they had to move it little to fit nicely. But the rest 90%, were very satisfied with the fitting status of the device.
- **Comfortability:** One only complained of sense of irritation during use of the device first night, which finished later on. And 95% said its use was comfortable and did not interfere with their sleep.
- **Sleeping apnea:** The two patients of sleeping apnea, one said during use of the device (Dr. Snore) he did not get any attack of sleeping apnea, as usually he suffer. The second one had two attacks first night but later on during the other four nights; he did not have any attack.
- **Confidence:** All the 20 were confident to advise their friends to buy the device, in reply to the last question (After 5 days of use are you confident to advise your friends to by Dr. Snore?).

Even the sample size was small, but it gives indication about its efficiency as preliminary study.

The plan is to carry out a similar (**feed back**) study on 5,000 snorers, by enclosing a questionnaire with each sold device and requesting the buyers to fill its two parts, before and after 5 days use and to return the completed forms to the pharmacies from where they bought the device, for analysis.

### ADVANTAGES OF THE INVENTION:

The present invention has the following advantages:
**1.** The present device is very effective in preventing snoring and alleviating sleeping apnea.
**2.** The present nasal device is very small in size, simple, easy and safe to use, washable and can be sterilized.
**3.** Present nasal device avoids its use in the sensitive mucous membrane and stable in its place despite movements of the user during sleep.

## Claims

1. A nasal device useful for prevention of snoring and sleeping apnea, wherein the said device comprising a stainless steel Skelton having one continuous stainless steel wire of unique design (as apparent in Figure 3), configured with the spring (1) having two prominent side knobs (2) and two resilient acute angles (6) to permit the abduction arms (4) and plates (7) to be worn (or inserted) inside the nose, wherein the said arms consisting of abduction plates (7) at its free end enabling its base resting on the columellae outside the nose and in continuity with the abduction arms by the said base arms (3), and the whole said device is coated with a thin layer of latex sponge or silicon to make it comfortable during use.

2. The nasal device as claimed in claim 1, wherein the stainless steel wire used is having a diameter of at least about 0.7mm.

3. The nasal device claimed in claim 1, wherein the abduction arms and plate's (together) length are in two sizes of about 1.6mm & about 1.4mm respectively, to fit in different nostrils.

4. The nasal device as claimed in claim 1, wherein the length of base arms are about 10 mm to facilitate controlling abduction arms.

5. The nasal device as claimed in claim 1, wherein the internal opening of the device enlarges by pressing of the side knobs.

6. The nasal device as claimed in claim 1, wherein the resilient acute angles have about 80 degree each.

7. The nasal device as claimed in claim 1, wherein the abduction plates at the free ends of the abduction arms, in a circle form about 3mm diameter.

8. The nasal device as claimed in claim 1, wherein the latex sponge and silicone coating used, in a thickness of at least about 1mm.

9. The nasal device substantially as herein described with reference to description and figures.
